# FASCICULE DE BREVET EUROPEEN

(11) **EP 0 558 370 B1**
(45) Date de publication et mention de la délivrance du brevet: **04.06.1997**
(21) Numéro de dépôt: 93400362.5
(22) Date de dépôt: 12.02.1993
(51) Int. Cl.: C07C 323/52, C07C 317/44, C07C 327/22, C07C 327/32, C08F 120/38

(54) **Nouveaux composés (méth)acryliques, leur procédé de préparation et leur application à la synthèse de nouveaux polymères**
Neue (Meth)Acrylverbindungen, Verfahren zu ihrer Herstellung und ihre Verwendung in der Synthese von neuen Polymeren
New (meth)acrylic compounds, process for their preparation and their use in the synthesis of new polymers

(30) Priorité: 17.02.1992 FR 9201748
(43) Date de publication de la demande: 01.09.1993
(73) Titulaire: ELF ATOCHEM S.A., 92800 Puteaux (FR)
(72) Inventeur: Curci, Michèle, F-57000 Metz (FR); Mieloszynski, Jean-Luc, F-57158 Montigny les Metz (FR); Paquer, Daniel, F-54500 Vandoeuvre (FR)
(74) Mandataire: Chaillot, Geneviève

(56) Documents cités:
- EP-A- 0 020 000
- EP-A- 0 367 577
- EP-A- 0 382 477
- EP-A- 0 463 947
- FR-A- 2 071 386
- US-A- 3 888 912
- CHEMICAL ABSTRACTS, vol. 114, no. 15, 15 avril 1991, Columbus, Ohio, US; abrégé no. 135867f, I. UEDA et al, page 52, colonne 2

## Description

La présente invention porte sur une nouvelle famille de composés (méth)acryliques, sur des procédés permettant d'obtenir ces composés, et sur l'application de ces derniers à la synthèse de nouveaux polymères.

Le brevet EP-A-0 382 477 décrit composés soufrés acryliques de type hydrocarbure.

L'invention vise à élargir la gamme disponible des composés (méth)acryliques, en particulier des composés (méth)acryliques soufrés, en proposant par ailleurs des procédés simples et efficaces pour préparer ces nouveaux composés, applicables à la préparation de nouveaux polymères et copolymères.

La présente invention a donc d'abord pour objet des composés (méth)acryliques représentés par la formule (I) suivante : 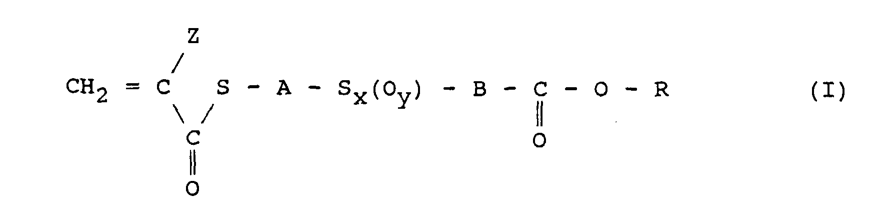 dans laquelle :
- Z représente H, un radical alkyle, linéaire ou ramifié;
- A et B représentent chacun indépendamment un radical alkylène, ce radical pouvant être substitué ;
- R représente H ou un métal alcalin, ou bien un radical alkyle, linéaire ou ramifié ;
- x vaut 0 ou 1 ; et
- y vaut 0, 1 ou 2, mais vaut 0 lorsque x = 0 ;
le radical A n'existant pas si x et y sont tous deux égaux à 0 ; B ne représentant pas méthylène éventuellement substitué quand x = y = O et R = alkyle.

Comme radicaux alkyle entrant dans la définition de Z ou R, on peut citer les radicaux méthyle, éthyle, propyle, tertiobutyle, n-butyle.

Le métal alcalin entrant dans la définition de R est choisi notamment parmi le sodium ou le potassium.

Des composés (méth)acryliques préférés sont ceux pour lesquels, dans la formule (I),
- Z représente H ou un radical méthyle ;
- A et B représentent chacun indépendamment un reste -(CH₂)ₙ-, n représentant un nombre entier de 1 à 20 ;
- R représente H, un métal alcalin ou un radical alkyle ; et
- x et y sont tels que définis ci-dessus.

Les composés représentés par les formules (Ia), (Ib) et (Ic) suivantes : 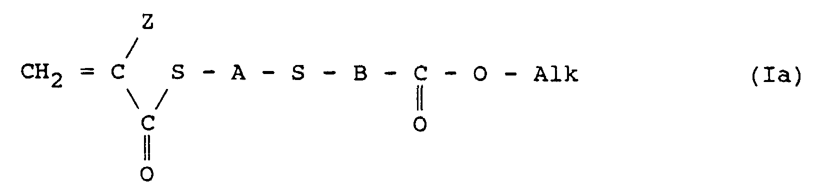 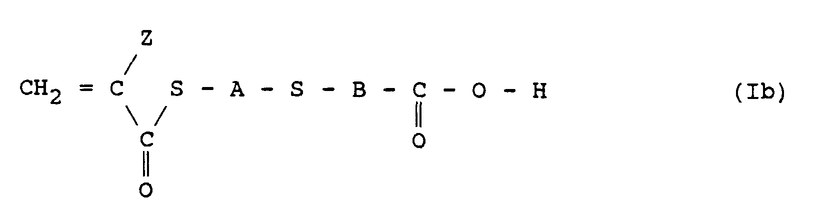 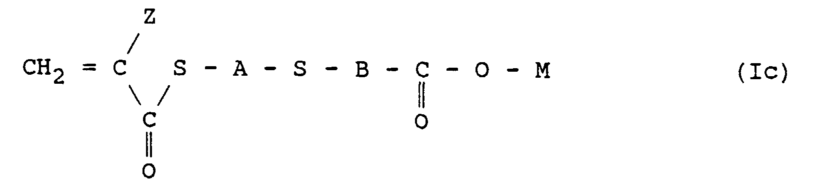 dans lesquelles :
- Z représente H, un radical alkyle, linéaire ou ramifié ;
- A et B représentent chacun indépendamment un radical alkylène éventuellement substitué ;
- M représente un métal alcalin, et Alk, un radical alkyle, linéaire ou ramifié,
peuvent être préparés par un procédé suivant lequel on fait réagir un composé représenté par la formule (II) suivante : 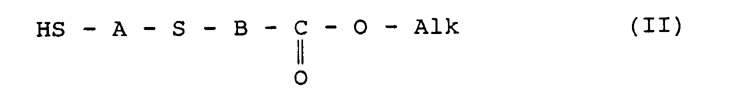 dans laquelle X, A, B et Alk sont tels que définis ci-dessus, avec un composé représenté par la formule (III) suivante : 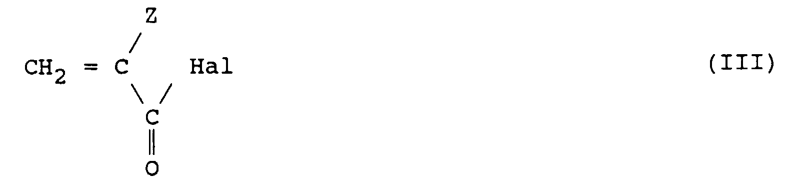 dans laquelle :
- Z est tel que défini ci-dessus ; et
- Hal représente un halogène, en particulier, chlore, brome, iode,
ce qui conduit à un composé de la formule (Ia) que l'on peut traiter par des ions H⁺, pour obtenir un acide de formule (Ib) que l'on peut salifier pour obtenir un sel de formule (Ic).

Le radical tertio-butyle pour Alk dans le composé (II) est préféré lorsque l'on souhaite obtenir la forme acide (Ib), car le radical tertio-butyle est celui le plus facilement éliminable.

La réaction des composés (II) et (III) est généralement conduite avec un léger excès molaire du composé (III) par rapport au composé (II), par exemple avec un rapport molaire composé (III)/composé (II) de 1,1/1, et dans un solvant, tel que le chloroforme, le tétrachlorure de carbone, le chlorure de méthylène, ou un solvant aromatique comme le toluène. La réaction est avantageusement conduite à une température de 0°C ou aux environs de 0°C pendant l'addition du composé (III), avec retour ultérieur à la température ambiante, et pendant une durée de l'ordre de quelques heures, par exemple d'environ 24 heures.

La réaction du composé de formule (II) et du composé de formule (III) est par ailleurs généralement effectuée en présence d'au moins un composé capable d'inhiber la polymérisation du composé de formule (III), utilisé, par exemple, à raison de 0,004% à 0,1% environ en poids sur la base du poids du composé de formule (III). Comme exemples d'inhibiteurs de polymérisation utilisables, on peut citer notamment la phénothiazine, l'éther monométhylique de l'hydroquinone, la N,N'-diéthyl-hydroxylamine, le nitrobenzène, le di-tertiobutylcatéchol, l'hydroquinone, le p-anilinophénol, le phosphite de di-(éthyl-2 hexyl)-octyl phényle, le bleu de méthylène et leurs mélanges en toutes proportions.

Pour obtenir l'acide (Ib), on traite le composé (Ia) par des ions H⁺, apportés par des acides organiques, comme l'acide formique, ou par des acides minéraux.

La présente invention concerne également, à titre d'intermédiaires de synthèse des composés de la formule (Ia) telle que définie ci-dessus, les composés représentés par la formule (II) : 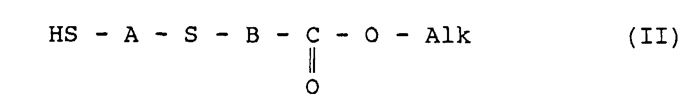 dans laquelle A, B et Alk sont tels que définis ci-dessus.

Pour préparer ces composés de la formule (II), on peut utiliser l'un des trois procédés suivants :

Conformément au premier procédé,
- on fait réagir avec l'acide nitrique un composé de la formule suivante (IV) :

   Hal - B - CH₂OH (IV)

   dans laquelle Hal représente un halogène, en particulier le chlore, le brome, l'iode, et notamment le chlore, et B est tel que défini ci-dessus ;
- on fait réagir le composé de formule (V) ainsi obtenu : 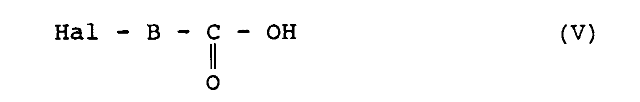 avec un composé de la formule (VI) suivante :

   Alk - OH (VI)

   dans laquelle Alk est tel que défini ci-dessus ;
- on fait réagir le composé de formule (VII) ainsi obtenu : 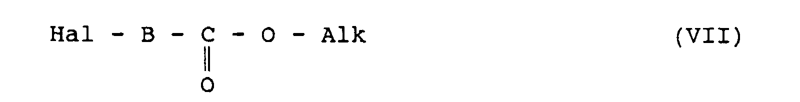 avec un composé de la formule (VIII) suivante :

   HS - A - SH (VIII)

   dans laquelle A est tel que défini ci-dessus.

Conformément au second procédé, on fait réagir un composé de la formule (IX) suivante : 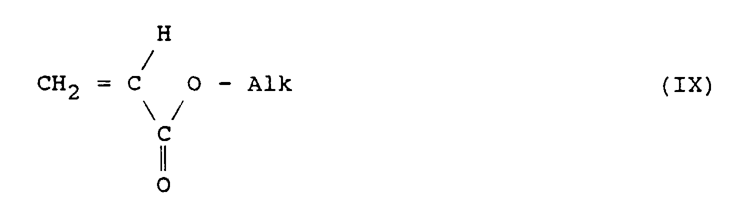 dans laquelle Alk est tel que défini ci-dessus,
avec un composé de la formule (VIII) telle que définie ci-dessus, ce qui conduit au composé de formule (IIa) :

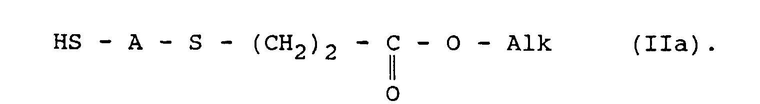

Conformément au troisième procédé,
- on fait réagir un composé de la formule (X) suivante : 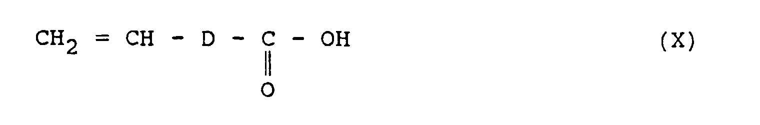 dans laquelle D a les mêmes significations que A ou B ci-dessus ;
   avec un composé de la formule (VI) telle que définie ci-dessus ; et
- on fait réagir le composé de la formule (XI) ainsi obtenu : 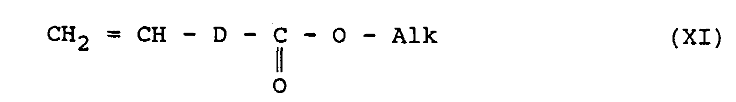 avec le composé de la formule (VIII) telle que définie ci-dessus, ce qui conduit à un composé de formule (IIb) :

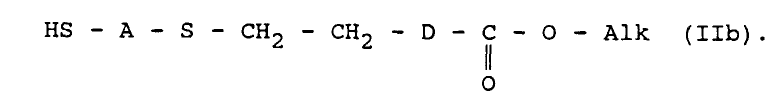

Les composés représentés par les formules (Id), (Ie) et (If) suivantes : 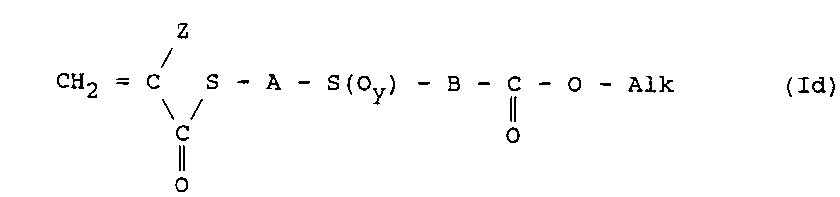 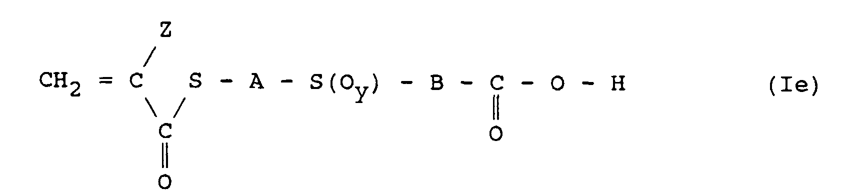 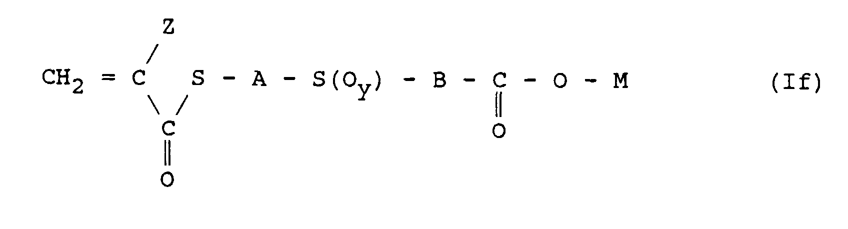 dans lesquelles :
- Z, A, B, Alk et M sont tels que définis ci-dessus ; et
- y vaut 1 ou 2 ;
sont préparés par le procédé consistant à faire réagir un composé de la formule (Ia) telle que définie ci-dessus avec un agent oxydant, le rapport molaire agent oxydant/composé (Ia) étant au moins égal à 1 et inférieur à 2 pour parvenir à un sulfoxyde (y=1) ; et au moins égal à 2 pour parvenir à une sulfone (y=2),
ce qui conduit à un composé de formule (Id), que l'on peut traiter par des ions H⁺, pour obtenir un acide de la formule (Ie), que l'on peut salifier pour obtenir un sel de la formule (If).

L'agent oxydant peut être l'eau oxygénée, de préférence, à une concentration comprise entre 5% et 50% environ ; un peracide organique, tel que l'acide peracétique, l'acide performique, l'oxygène, l'air, le diméthyl sulfoxyde, le periodate de sodium ou l'acide métachloroperbenzoïque.

Les composés représentés par les formules (Ig), (Ih) et (Ii) suivantes : 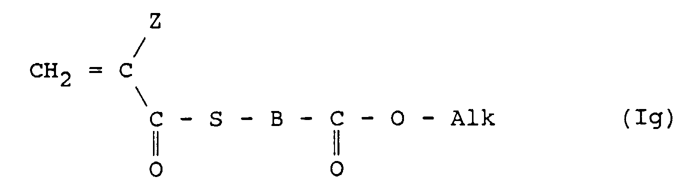 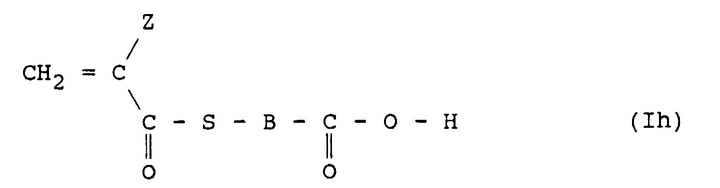 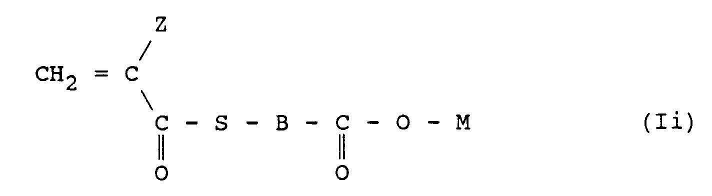 dans lesquelles Z, B, Alk et M sont tels que définis ci-dessus,
peuvent être préparés par le procédé consistant à faire réagir un composé de la formule (VII) telle que définie ci-dessus sur un composé de la formule (XII) suivante : 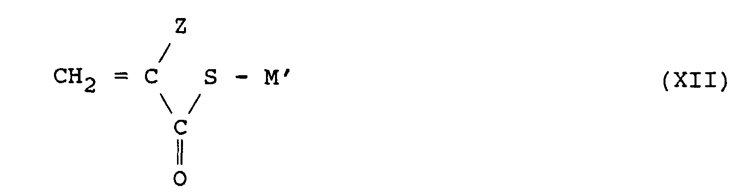 dans laquelle :
- Z est tel que défini ci-dessus ; et
- M' représente un métal alcalin, par exemple, le lithium, le sodium ou le potassium,
ce qui conduit à un composé de formule (Ig), que l'on peut traiter par des ions H⁺ pour obtenir l'acide de formule (Ih), que l'on peut salifier pour obtenir le sel de formule (Ii).

La réaction du (méth)acrylate de formule (XII) et du composé de formule (VII) est effectuée à une température qui dépend du point d'ébullition du composé de formule (VII), en milieu solvant tel que l'acétonitrile, et en présence d'au moins un agent de transfert de phase, utilisé par exemple à raison de 5% à 30% environ en moles par rapport au (méth)acrylate de formule (XII). Comme exemples d'agents de transfert de phase utilisables conformément à ce procédé, on peut citer notamment les sels d'ammonium quaternaires, les sels de phosphonium quaternaires, les sels d'arsénium quaternaires, les éthers de polyéthylène glycols, les complexants macrohétérocycliques de la famille des cryptants aprotiques tels que ceux décrits dans FR-A-2 398 079, et les complexants macrocycliques non azotés, tels que ceux décrits dans US-A-3 687 978.

Le (méth)acrylate de formule (XII) et le composé halogéné sont généralement, dans le procédé selon l'invention, utilisés en proportions telles que le rapport (méth)acrylate (XII)/composé (VII) soit au moins égal à 1 et de préférence compris entre 1 et 1,6 environ. La durée de la réaction est généralement comprise entre 30 minutes et 20 heures environ.

La présente invention concerne enfin l'application des nouveaux composés acryliques décrits précédemment à la constitution de nouveaux polymères et copolymères. Plus précisément la présente invention concerne des polymères et copolymères comprenant au moins un motif dérivé d'au moins un composé (méth)acrylique de formule (I). De tels (co)polymères peuvent en outre comprendre au moins un motif dérivé d'au moins un comonomère copolymérisable avec ledit composé (méth)acrylique.

## Revendications

1. Composés (méth)acryliques représentés par la formule (I) : 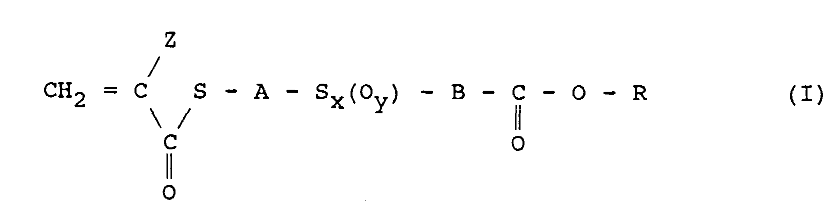 dans laquelle :
- Z représente H, un radical alkyle, linéaire ou ramifié;
- A et B représentent chacun indépendamment un radical alkylène, ce radical pouvant être substitué ;
- R représente H ou un métal alcalin, ou bien un radical alkyle, linéaire ou ramifié ;
- x vaut 0 ou 1 ; et
- y vaut 0, 1 ou 2 mais vaut 0 lorsque x = 0 ;
le radical A n'existant pas si x et y sont tous deux égaux à 0 ; B ne représentant pas méthylène éventuellement substitué quand x = y = 0 et R = alkyle.

2. Procédé de fabrication d'un composé acrylique représenté par la formule (I) telle que définie à la revendication 1 caractérisé par le fait qu'il comprend :
- la réaction d'un composé de la formule (II) : 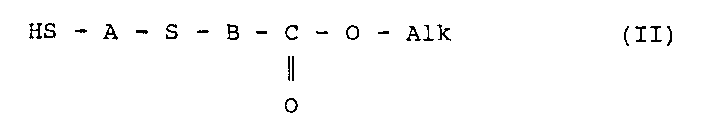
- dans laquelle A et B sont tels que définis à la revendication 1 et Alk représente un radical alkyle linéaire ou ramifié,
avec un composé de la formule (III) : 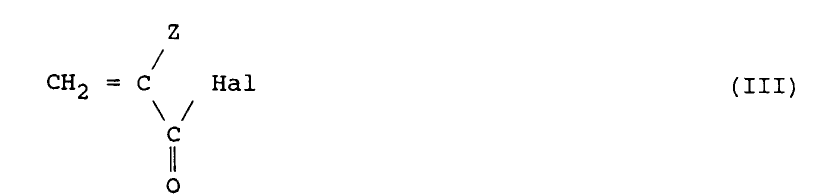
- dans laquelle Z est tel que défini à la revendication 1 et Hal représente un halogène,
de façon à obtenir un composé (I) dans lequel x = 1, y = 0 et R = alkyle (composé (Ia)) ;
- le traitement dudit composé (Ia) par des ions H⁺ si l'on souhaite obtenir un composé (I) dans lequel x = 1, y = 0 et R = H (composé (Ib)) ;
- la conversion dudit composé (Ib) en un sel si l'on souhaite obtenir un composé (I) dans lequel x = 1, y = 0 et R = métal alcalin (composé (Ic)) ;
- la réaction dudit composé (Ia) avec un agent oxydant si l'on souhaite obtenir un composé (I) dans lequel x = 1, y = 1 ou 2 et R = alkyle (composé (Id)), le rapport molaire agent oxydant/composé (Ia) étant au moins égal à 1 et inférieur à 2, pour obtenir un sulfoxyde (y = 1), et au moins égal à 2, pour obtenir une sulfone (y = 2) ;
- le traitement dudit composé (Id) par des ions H⁺ si l'on souhaite obtenir un composé (I) dans lequel x = 1, y = 1 ou 2 et R = H (composé (Ie)) ;
- la conversion dudit composé (Ie) en un sel si l'on souhaite obtenir un composé (I) dans lequel x = 1, y = 1 ou 2 et R = métal alcalin (composé (If)) ; ou qu'il comprend :
- la réaction d'un composé de la formule (XII) : 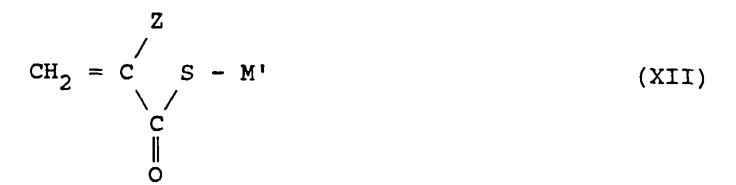
- dans laquelle Z est tel que défini à la revendication 1 et M' représente un métal alcalin,
avec un composé de la formule (VII) : 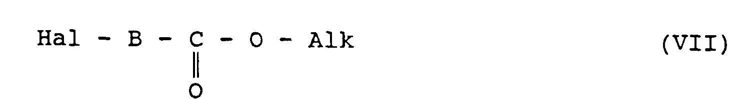
- dans laquelle Hal, B et Alk sont tels que définis ci-dessus,
pour obtenir un composé (I) dans lequel x = y = 0 et R = alkyle (composé (Ig)) ;
- le traitement dudit composé (Ig) par des ions H⁺ si l'on souhaite obtenir un composé (I) dans lequel x = y = 0 et R représente H (composé (Ih)) ;
- la conversion dudit composé (Ih) en un sel si l'on souhaite obtenir un composé (I) dans lequel x = y = 0 et R = métal alcalin (composé (Ii)).

3. Procédé selon la revendication 2, caractérisé par le fait que l'on prépare le composé de formule (II) par :
- la réaction avec l'acide nitrique d'un composé de la formule (IV) :
Hal - B - CH₂OH (IV)
- dans laquelle Hal représente un halogène, et B est tel que défini à la revendication 2,
pour obtenir un composé de formule (V) : 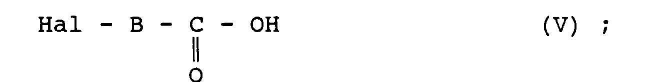
- la réaction du composé de formule (V) ainsi obtenu avec un composé de la formule (VI) :
Alk - OH (VI)
- dans laquelle Alk est tel que défini à la revendication 2, pour obtenir un composé de formule (VII) : 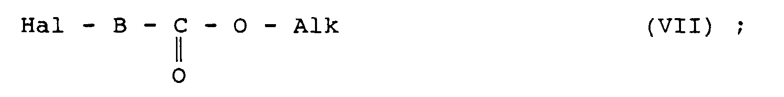 et
- la réaction du composé de formule (VII) ainsi obtenu avec un composé de la formule (VIII) suivante :
HS - A - SH (VIII)
- dans laquelle A est tel que défini à la revendication 2.

4. Procédé selon la revendication 2, caractérisé par le fait que l'on prépare un composé de formule (II) dans laquelle B représente -(CH₂)₂-, et A et Alk sont tels que définis à la revendication 2, en faisant réagir un composé de la formule (IX) : 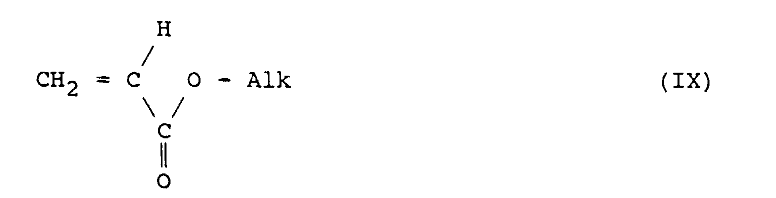
- dans laquelle Alk est tel que défini ci-dessus,
avec un composé de la formule (VIII) telle que définie à la revendication 3.

5. Procédé selon la revendication 2, caractérisé par le fait que l'on prépare un composé de formule (II) dans laquelle B représente -CH₂-CH₂-D-, D représentant un radical alkylène, substitué ou non,
- en faisant réagir un composé de la formule (X) : 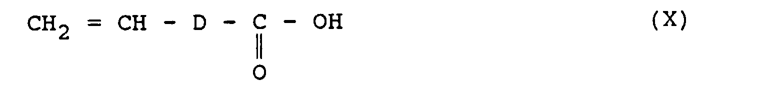
- dans laquelle D est tel que défini ci-dessus ;
avec un composé de la formule (VI) telle que définie à la revendication 3,
afin d'obtenir un composé de la formule (XI) : 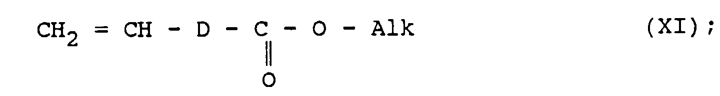 et
- en faisant réagir ledit composé (XI) avec le composé de la formule (VIII) telle que définie à la revendication 3.

6. Polymère comprenant au moins un motif d'au moins un composé de la formule (I) telle que définie à la revendication 1.

## Patentansprüche

1. (Meth-)Acrylverbindungen der Formel (I) 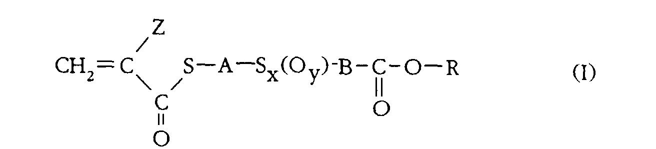 in der:
- Z Wasserstoff oder einen linearen oder verzweigten Alkylrest bezeichnet;
- A und B jeweils unabhängig voneinander einen Alkylenrest bezeichnen, der substituiert sein kann;
- R Wasserstoff oder ein Alkalimetall oder auch einen linearen oder verzweigten Alkylrest darstellt;
- x 0 oder 1 ist; und
- y 0, 1 oder 2 ist, jedoch 0 beträgt, wenn x = 0;
wobei der Rest A nicht vorhanden ist, wenn x und y beide gleich 0 sind; B kein gegebenenfalls substituiertes Methylen darstellt, wenn x = y = 0 und R = Alkyl.

2. Verfahren zur Herstellung einer (Meth-)Acrylverbindung der Formel (I) gemäß Anspruch 1, dadurch gekennzeichnet, daß es umfaßt:
- Reaktion einer Verbindung der Formel (II) 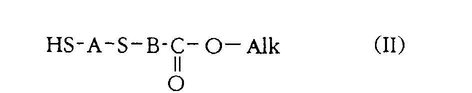
- in der A und B so wie in Anspruch 1 definiert sind und Alk einen linearen oder verzweigten Alkylrest bezeichnet;
mit einer Verbindung der Formel (III) 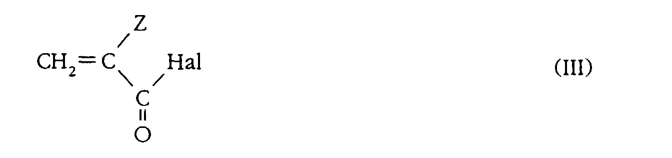
- in der Z wie in Anspruch 1 definiert ist und Hal ein Halogen darstellt,
so daß man eine Verbindung (I) erhält, in der x = 1, y = 0 und R = Alkyl (Verbindung (Ia));
- Behandlung der Verbindung (Ia) durch H⁺-Ionen, wenn man eine Verbindung (I) erhalten möchte, in der x = 1, y = 0 und R = H (Verbindung (Ib));
- Umsetzung der Verbindung (Ib) zu einem Salz, wenn man eine Verbindung (I) erhalten möchte, in der x = 1, y = 0 und R = Alkalimetall (Verbindung (Ic));
- Reaktion der Verbindung (Ia) mit einem Oxidationsmittel, wenn man eine Verbindung (I) erhalten möchte, in der x = 1, y = 1 oder 2 und R = Alkyl (Verbindung (Id)), wobei das Molverhältnis von Oxidationsmittel/Verbindung (Ia) mindestens gleich 1 und kleiner als 2 ist, um ein Sulfoxid (y = 1) zu erhalten, und mindestens gleich 2 ist, um ein Sulfon (y = 2) zu erhalten;
- Behandlung der Verbindung (Id) durch H⁺-Ionen, wenn man eine Verbindung (I) erhalten möchte, in der x = 1, y = 1 oder 2 und R=H (Verbindung (Ie));
- Umsetzung der Verbindung (Ie) zu einem Salz, wenn man eine Verbindung (I) erhalten möchte, in der x = 1, y = 1 oder 2 und R = Alkalimetall (Verbindung (If)); oder
das umfaßt:
- Reaktion einer Verbindung der Formel (XII) 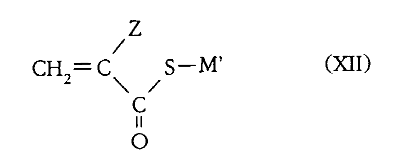
- in der Z wie in Anspruch 1 definiert ist und M' ein Alkalimetall darstellt mit einer Verbindung der Formel (VII) 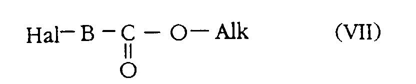
- in der Hal, B und Alk wie oben definiert sind,
um eine Verbindung (I) zu erhalten, in der x = y = 0 und R = Alkyl ist (Verbindung (Ig));
- Behandlung der Verbindung (Ig) durch H⁺-Ionen, wenn man eine Verbindung (I) erhalten möchte, in der x = y = 0 und R = H (Verbindung (Ih));
- Umsetzung der Verbindung (I^{h}) zu einem Salz, wenn man eine Verbindung (I) erhalten möchte, in der x = y = 0 und R = Alkalimetall (Verbindung (Ii)).

3. Verfahren nach Anspruch 2, dadurch gekennzeichnet, daß man die Verbindung der Formel (II) herstellt durch
- Reaktion mit Salpetersäure einer Verbindung der Formel (IV)
Hal-B-CH₂OH (IV)
- in der Hal ein Halogen darstellt und B wie in Anspruch 2 definiert ist,
um eine Verbindung der Formel (V) zu erhalten 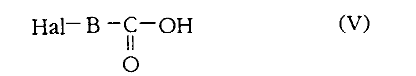
- Reaktion der so erhaltenen Verbindung der Formel (V) mit einer Verbindung der Formel (VI)
Alk - OH (VI)
- in der Alk wie in Anspruch 2 definiert ist,
um eine Verbindung der Formel (VII) zu erhalten 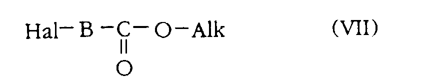 und
- Reaktion der so erhaltenen Verbindung der Formel (VII) mit einer Verbindung der Formel (VIII)
HS - A- SH (VIII)
- in der A wie in Anspruch 2 definiert ist.

4. Verfahren nach Anspruch 2, dadurch gekennzeichnet, daß man eine Verbindung der Formel (II) herstellt, in der B den Rest -(CH₂)₂- darstellt und A und Alk wie in Anspruch 2 definiert sind, wobei man eine Verbindung der Formel (IX) 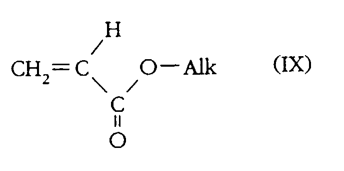
- in der Alk wie oben definiert ist
mit einer Verbindung der Formel (VIII), die wie in Anspruch 3 definiert ist, reagieren läßt.

5. Verfahren nach Anspruch 2, dadurch gekennzeichnet, daß man eine Verbindung der Formel (II) herstellt, in der B den Rest -CH₂-CH₂-D- darstellt, wobei D einen gegebenenfalls substituierten Alkylenrest darstellt,
- indem man eine Verbindung der Formel (X) 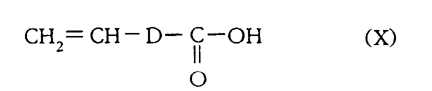
- in der D wie oben definiert ist,
mit einer Verbindung der Formel (VI), die wie in Anspruch 3 definiert ist, reagieren läßt,
um eine Verbindung der Formel (XI) zu erhalten 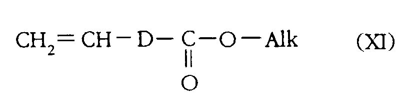 und
- indem man die Verbindung (XI) mit der Verbindung der Formel (VIII), die wie in Anspruch 3 definiert ist, reagieren läßt.

6. Polymer, enthaltend mindestens eine Einheit mindestens einer Verbindung der Formel (I) gemäß Anspruch 1.

## Claims

1. (Meth)acrylic compounds represented by the formula (I) : 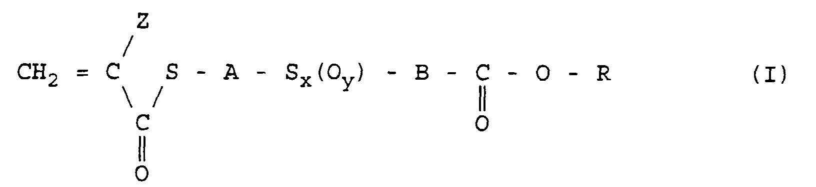 in which :
- Z denotes H, a linear or branched alkyl radical ;
- each of A and B independently denotes an alkylene radical, it being possible for this radical to be substituted ;
- R denotes H or an alkali metal, or else a linear or branched alkyl radical ;
- x has the value of 0 or 1 ; and
- y has the value of 0, 1 ou 2, but has the value 0 when x = 0 ;
the radical A not existing if x and y are both equal to 0 ; B not denoting optionally substituted methylene when x = y = 0 and R = alkyl.

2. Process for the manufacture of an acrylic compound represented by the formula (I) as defined in Claim 1, characterized by the fact that it comprises :
- reacting a compound of formula (II) : 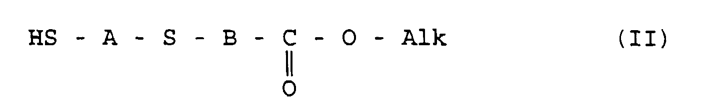
- in which A and B are as defined in Claim 1 and Alk denotes a linear or branched alkyl radical,
with a compound of formula (III) : 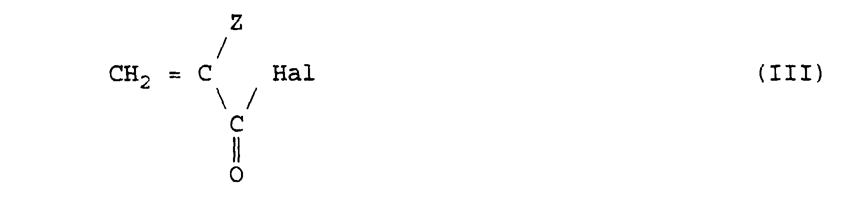
- in which Z is as defined in Claim 1 and Hal denotes a halogen,
in order to obtain a compound (I) in which x = 1, y = 0 and R = alkyl (compound (Ia)) ;
- treating said compound (Ia) with ions H⁺ if it is desired to obtain a compound (I) in which x = 1, y = 0 and R = H (compound (Ib)) ;
- converting said compound (Ib) into a salt if it is desired to obtain a compound (I) in which x = 1, y = 0 and R = alkali metal (compound (Ic)) ;
- reacting said compound (Ia) with an oxidising agent if it is desired to obtain a compound (I) in which x = 1, y = 1 or 2 and R = alkyl (compound (Id), the molar ratio oxidising agent/compound (Ia) being at least equal to 1 and lower than 2 in order to obtain a sulphoxide (y = 1), and at least equal to 2 in order to obtain a sulphone (y = 2) ;
- treating said compound (Id) with H⁺ ions if it is desired to obtain a compound (I) in which x = 1, y = 1 or 2 and R = H (compound (Ie)) ;
- converting said compound (Ie) into a salt if it is desired to obtain a compound (I) in which x = 1, y = 1 or 2 and R = alkali metal (compound (If)) ; or
that it comprises :
- reacting a compound of the formula (XII) : 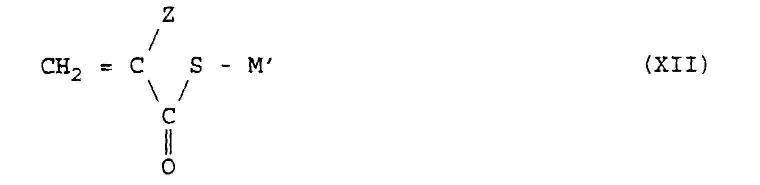
- in which Z is as defined in Claim 1 and M' denotes an alkali metal,
with a compound of the formula (VII) : 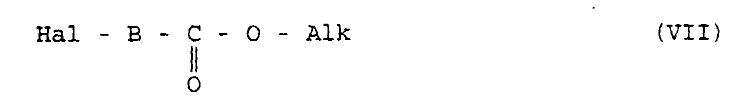
- in which Hal, B and Alk are as defined above,
in order to obtain a compound (I) in which x = y = 0 and R = alkyl (compound (Ig)) ;
- treating said compound (Ig) with H⁺ ions if it is desired to obtain a compound (I) in which x = y = 0 and R denotes H (compound (Ih)) ;
- converting said compound (Ih) into a salt if it is desired to obtain a compound (I) in which x = y = 0 and R = alkali metal (compound (Ii)).

3. Process according to claim 2, characterized by the fact that the compound of the formula (II) is prepared by :
- reacting a compound of the formula (IV) :
Hal - B - CH₂OH (IV)
- in which Hal denotes a halogen, and B is as defined in claim 2,
with nitric acid in order to obtain a compound of the formula (V) : 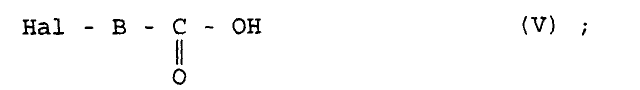
- reacting the compound of the formula (V) thus obtained with a compound of the formula (VI) :
Alk - OH (VI)
- in which Alk is as defined in Claim 2,
in order to obtain a compound of the formula (VII) : 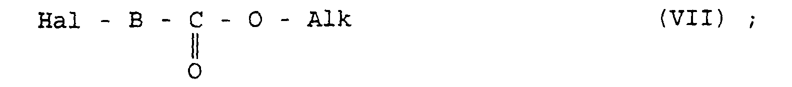 and
- reacting the compound of the formula (VII) thus obtained with a compound of the following formula (VIII) :
HS - A - SH (VIII)
- in which A is as defined in Claim 2.

4. Process according to Claim 2, characterized by the fact that a compound of the formula (II) in which B denotes -(CH₂)₂- and A and Alk are as defined in Claim 2, is prepared by reacting a compound of the formula (IX) : 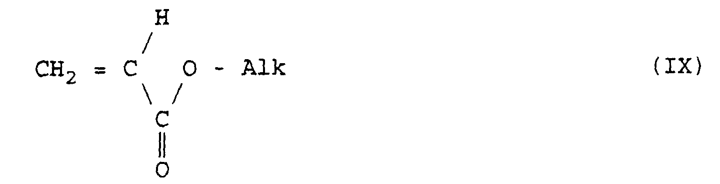
- in which Alk is as defined above,
with a compound of the formula (VIII) as defined in Claim 3.

5. Process according to Claim 2, characterized by the fact that a compound of the formula (II) in which B denotes -CH₂-CH₂-D, D denoting a substituted or not substituted alkylene radical, is prepared,
- by reacting a coumpound of the formula (X) : 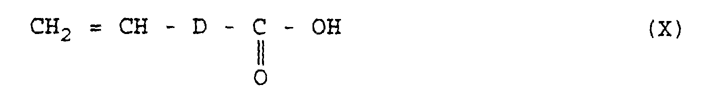
- in which D is as defined above,
with a compound of the formula (VI) as defined in Claim 3,
in order to obtain a compound of the formula (XI) : 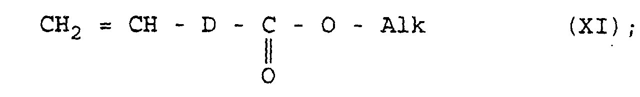 and
- by reacting said compound (XI) with the compound of the formula (VIII) as defined in Claim 3.

6. Polymer containing at least one unit of at least one compound of the formula (I) as defined in Claim 1.
